# EUROPEAN PATENT SPECIFICATION

(11) **EP 3 542 819 B1**
(45) Date of publication and mention of the grant of the patent: **18.08.2021**
(21) Application number: 19173426.8
(22) Date of filing: 12.05.2014
(51) Int. Cl.: A61K 39/39, A61K 39/008, A61K 39/27

(54) **NOVEL VACCINE COMPOSITIONS COMPRISING IMMUNOSTIMULATORY OLIGONUCLEOTIDES**
NEUARTIGE IMPFSTOFFZUSAMMENSETZUNGEN MIT IMMUNSTIMULIERENDEN OLIGONUKLEOTIDEN
NOUVELLES COMPOSITIONS DE VACCIN COMPRENANT DES OLIGONUCLÉOTIDES IMMUNOSTIMULATEURS

(30) Priority: 14.05.2013 US 201361823189 P
(43) Date of publication of application: 25.09.2019
(62) Divisional of application: 14729191.8
(73) Proprietor: Zoetis Services LLC, Parsippany, NJ 07054 (US)
(72) Inventor: Dominowski, Paul Joseph, Kalamazoo, MI 49007 (US); Rai, Sharath K., Kalamazoo, MI 49007 (US); Sly, Laurel Mary, Kalamazoo, MI 49007 (US); Cook, Corey Patrick, Kalamazoo, MI 49007 (US); Mwangi, Duncan, Kalamazoo, MI 49007 (US); Foss, Dennis L., Kalamazoo, MI 49007 (US); Krebs, Richard Lee, Kalamazoo, MI 49007 (US)
(74) Representative: Mannion, Sally Kim

(56) References cited:
- WO-A1-2011/148356
- WO-A2-2008/068638
- R. PAILLOT ET AL: "Equine Herpes Virus-1: Virus, Immunity and Vaccines", THE OPEN VETERINARY SCIENCE JOURNAL, vol. 2, no. 1, 1 January 2008 (2008-01-01) , pages 68-91, XP055604426, ISSN: 1874-3188, DOI: 10.2174/1874318808002010068
- CHRISTIAN BODE ET AL: "CpG DNA as a vaccine adjuvant", EXPERT REVIEW OF VACCINES, vol. 10, no. 4, 1 April 2011 (2011-04-01), pages 499-511, XP055138146, ISSN: 1476-0584, DOI: 10.1586/erv.10.174
- EVA WATTRANG ET AL: "Cytokine production and proliferation upon in vitro oligodeoxyribonucleotide stimulation of equine peripheral blood mononuclear cells", VETERINARY IMMUNOLOGY AND IMMUNOPATHOLOGY, ELSEVIER BV, AMSTERDAM, NL, vol. 146, no. 2, 9 February 2012 (2012-02-09), pages 113-124, XP028405705, ISSN: 0165-2427, DOI: 10.1016/J.VETIMM.2012.02.004 [retrieved on 2012-02-16]
- SEONG K. KIM ET AL: "Intranasal treatment with CpG-B oligodeoxynucleotides protects CBA mice from lethal equine herpesvirus 1 challenge by an innate immune response", ANTIVIRAL RESEARCH, vol. 169, 25 June 2019 (2019-06-25), page 104546, XP055604382, NL ISSN: 0166-3542, DOI: 10.1016/j.antiviral.2019.104546

## Description

### FIELD OF THE INVENTION

This invention relates to novel adjuvants for enhancing immune response to Equine Horse Virus vaccines.

### BACKGROUND

Equine herpes virus is a major equine pathogen responsible for viral-induced abortion neurological disease such as paresis, infections of the upper respiratory tract, and neonatal foal disease (NFD). NFD results from close to term transplacental infection of fetuses, which are born weak with severe respiratory disease and some with jaundice due to liver infection by EHV-1. These animals usually die within a few days after birth. Equine rhinopneumonitis virus (EHV-4) is the major cause of acute respiratory tract disease ("rhinopneumonitis") and infects most horses during their first two years of life. Rhinopneumonitis is characterized by fever, anorexia, and profuse serous nasal discharge that later becomes mucopurulent. On rare occasions EHV4 infection causes abortion in pregnant mares. Furthermore, EHV1 and EHV4 establish persistent, lifelong latent infections. Upon reactivation the viruses cause recurrent disease, accompanied by virus shedding and transmission to other animals.

Control of equine herpes virus infection and their diseases remain inadequate, in particular against EHV1 mediated abortions, paresis and neonatal foal disease resulting from close to term transplacental infection of foetus. Although inactivated as well as modified live vaccines are available, neither vaccine appears to block infection sufficiently, nor do they prevent the establishment of latency by wild-type virus. Hence there is a great need for safe vaccines with improved protection against field infections of these viruses, particularly against infections caused by EHV1.

R. Paillot et al, The Open Veterinary Science Journal, vol. 2, no. 1, 1 Jan 2008 pages 68-91, discloses many vaccines for EHV (p.79 onwards). Bode et al (Expert Review of Vaccines 2011; 10(4) 499-511) discuses the potential of different types of CpGs as adjuvants. Bode is silent with regard to the use of SP oil and EHV. Wattrang et al discloses the effects of different CpG classes on type I interferons (IFN), IFN-y, tumor necrosis factor-a (TNF-o) and transforming growth factor-p (TGFP), and proliferation of equine peripheral blood mononuclear cells (PBMC). Davis et al (WO2011/148356) discloses a combination of CpG oligonucleotides and cholesterol. Debelak et al (WO2008/068638) discloses P-class CpG oligonucleotides having 5' base substituted with lipophilic analogs but does not mention EHV or SP oil.

Numerous treatments have been described but none is fully satisfactory due to toxicity of the treatment itself or a tendency for the animal to relapse.

Accordingly, new vaccine formulations are needed to protect domestic animals against EHV infections.

### SUMMARY OF INVENTION

The instant invention addresses drawbacks of the art by providing, in one aspect, a vaccine composition comprising an equine herpes virus (EHV) antigen component, wherein the antigen component comprises an EHV-1 antigen, and an adjuvant component, wherein the adjuvant component comprises a P-class CpG immunostimulatory oligonucleotide and SP oil wherein the SP oil comprises 2-20% v/v of the vaccine composition. The SP oil comprises about 2.5% w/v polyoxyethylene-polyoxypropylene block copolymer, about 5% w/v squalane, and about 0.2% w/v polyoxyethylene sorbitan monooleate.

In a set of embodiments, the P-class immunostimulatory oligonucleotide, which may be modified and/or stabilized, is present in the amount of 20-500 µg per dose, e.g., 100-250 µg per dose.

In another embodiment, the SP oil comprises about 5% to about 10% of the vaccine composition.

In certain embodiments of the invention, the vaccine formulation is non-liposomal.

### DETAILED DESCRIPTION OF SELECTED EMBODIMENTS

The following non-limiting definitions are provided for a better understanding of the invention:

"About" or "approximately," when used in connection with a measurable numerical variable, refers to the indicated value of the variable and to all values of the variable that are within the experimental error of the indicated value (e.g., within the 95% confidence interval for the mean) or within 10 percent of the indicated value, whichever is greater, unless about is used in reference to time intervals in weeks where "about 3 weeks," is 17 to 25 days, and about 2 to about 4 weeks is 10 to 40 days.

"Adjuvant" means any substance that increases the humoral or cellular immune response to an antigen. Adjuvants are generally used to accomplish two objectives: The slow the release of antigens from the injection site, and the stimulation of the immune system.

"Antibody" refers to an immunoglobulin molecule that can bind to a specific antigen as the result of an immune response to that antigen. Immunoglobulins are serum proteins composed of "light" and "heavy" polypeptide chains having "constant" and "variable" regions and are divided into classes (e.g., IgA, IgD, IgE, IgG, and IgM) based on the composition of the constant regions.

"Antigen" or "immunogen" refers to any substance that stimulates an immune response. The term includes killed, inactivated, attenuated, or modified live bacteria, viruses, or parasites. The term antigen also includes polynucleotides, polypeptides, recombinant proteins, synthetic peptides, protein extract, cells (including tumor cells), tissues, polysaccharides, or lipids, or fragments thereof, individually or in any combination thereof. The term antigen also includes antibodies, such as anti-idiotype antibodies or fragments thereof, and to synthetic peptide mimotopes that can mimic an antigen or antigenic determinant (epitope).

"Bacterin" means a suspension of one or more killed bacteria which may be used as a component of a vaccine or immunogenic composition.

"Buffer" means a chemical system that prevents change in the concentration of another chemical substance, e.g., proton donor and acceptor systems serve as buffers preventing marked changes in hydrogen ion concentration (pH). A further example of a buffer is a solution containing a mixture of a weak acid and its salt (conjugate base) or a weak base and its salt (conjugate acid).

"Cellular immune response" or "cell mediated immune response" is one mediated by T-lymphocytes or other white blood cells or both, and includes the production of cytokines, chemokines and similar molecules produced by activated T-cells, white blood cells, or both.

"Consisting essentially" as applied to the adjuvant formulations refers to formulation which does not contain unrecited additional adjuvanting or immunomodulating agents in the amounts at which said agent exert measurable adjuvanting or immunomodulating effects.

"Dose" refers to a vaccine or immunogenic composition given to a subject. A "first dose" or "priming vaccine" refers to the dose of such a composition given on Day 0. A "second dose" or a "third dose" or an "annual dose" refers to an amount of such composition given subsequent to the first dose, which may or may not be the same vaccine or immunogenic composition as the first dose.

"Humoral immune response" refers to one that is mediated by antibodies.

"Immune response" in a subject refers to the development of a humoral immune response, a cellular immune response, or a humoral and a cellular immune response to an antigen. Immune responses can usually be determined using standard immunoassays and neutralization assays, which are known in the art.

"Immunologically protective amount" or "immunologically effective amount" or "effective amount to produce an immune response" of an antigen is an amount effective to induce an immunogenic response in the recipient. The immunogenic response may be sufficient for diagnostic purposes or other testing, or may be adequate to prevent signs or symptoms of disease, including adverse health effects or complications thereof, caused by infection with a disease agent. Either humoral immunity or cell-mediated immunity or both may be induced. The immunogenic response of an animal to an immunogenic composition may be evaluated, e.g., indirectly through measurement of antibody titers, lymphocyte proliferation assays, or directly through monitoring signs and symptoms after challenge with wild type strain, whereas the protective immunity conferred by a vaccine can be evaluated by measuring, e.g., reduction in clinical signs such as mortality, morbidity, temperature number, overall physical condition, and overall health and performance of the subject. The immune response may comprise, without limitation, induction of cellular and/or humoral immunity.

"Immunogenic" means evoking an immune or antigenic response. Thus an immunogenic composition would be any composition that induces an immune response.

"Immunostimulatory molecule" refers to a molecule that generates an immune response.

"Lipids" refers to any of a group of organic compounds, including the fats, oils, waxes, sterols, and triglycerides that are insoluble in water but soluble in nonpolar organic solvents, are oily to the touch, and together with carbohydrates and proteins constitute the principal structural material of living cells.

"Pharmaceutically acceptable" refers to substances, which are within the scope of sound medical judgment, suitable for use in contact with the tissues of subjects without undue toxicity, irritation, allergic response, and the like, commensurate with a reasonable benefit-to-risk ratio, and effective for their intended use.

"Reactogenicity" refers to the side effects elicited in a subject in response to the administration of an adjuvant, an immunogenic, or a vaccine composition. It can occur at the site of administration, and is usually assessed in terms of the development of a number of symptoms. These symptoms can include inflammation, redness, and abscess. It is also assessed in terms of occurrence, duration, and severity. A "low" reaction would, for example, involve swelling that is only detectable by palpitation and not by the eye, or would be of short duration. A more severe reaction would be, for example, one that is visible to the eye or is of longer duration.

"Room Temperature" means a temperature from 18 to 25°C.

"Saponin" refers to a group of surface-active glycosides of plant origin composed of a hydrophilic region (usually several sugar chains) in association with a hydrophobic region of either steroid or triterpenoid structure.

"Steroids" refers to any of a group of organic compounds belonging to biochemical class of lipids, which are easily soluble in organic solvents and slightly soluble in water. Steroids comprise a four-fused ring system of three fused cyclohexane (six-carbon) rings plus a fourth cyclopentane (five-carbon) ring.

"Sterols" refers to compounds in animals which are biologically produced from terpenoid precursors. They comprise a steroid ring structure, having a hydroxyl (OH) group, usually attached to carbon-3. The hydrocarbon chain of the fatty-acid substituent varies in length, usually from 16 to 20 carbon atoms, and can be saturated or unsaturated. Sterols commonly contain one or more double bonds in the ring structure and also a variety of substituents attached to the rings. Sterols and their fatty-acid esters are essentially water insoluble.

"Subject" refers to any animal for which the administration of an adjuvant composition is desired. It includes mammals and non-mammals, including primates, livestock, companion animals, laboratory test animals, captive wild animals, aves (including in ova), reptiles, and fish. Thus, this term includes but is not limited to monkeys, humans, swine; cattle, sheep, goats, equines, mice, rats, guinea pigs, hamsters, rabbits, felines, canines, chickens, turkeys, ducks, other poultry, frogs, and lizards.

"Therapeutically effective amount" refers to an amount of an antigen or vaccine that would induce an immune response in a subject receiving the antigen or vaccine which is adequate to prevent or reduce signs or symptoms of disease, including adverse health effects or complications thereof, caused by infection with a pathogen, such as a virus or a bacterium. Humoral immunity or cell-mediated immunity or both humoral and cell-mediated immunity may be induced. The immunogenic response of an animal to a vaccine may be evaluated, e.g., indirectly through measurement of antibody titers, lymphocyte proliferation assays, or directly through monitoring signs and symptoms after challenge with wild type strain. The protective immunity conferred by a vaccine can be evaluated by measuring, e.g., reduction in clinical signs such as mortality, morbidity, temperature number, overall physical condition, and overall health and performance of the subject. The amount of a vaccine that is therapeutically effective may vary depending on the particular adjuvant used, the particular antigen used, or the condition of the subject, and can be determined by one skilled in the art.

"Treating" refers to preventing a disorder, condition, or disease to which such term applies, or to preventing or reducing one or more symptoms of such disorder, condition, or disease.

"Treatment" refers to the act of "treating" as defined above.

"Triterpeniods" refers to a large and diverse class of naturally occurring organic molecules, derived from six five-carbon isoprene (2-methyl-1,3-butadiene) units, which can be assembled and modified in thousands of ways. Most are multicyclic structures which differ from one another in functional groups and in their basic carbon skeletons. These molecules can be found in all classes of living things.

"Vaccine" refers to a composition that includes an antigen, as defined herein. Administration of the vaccine to a subject results in an immune response, generally against one or more specific diseases. The amount of a vaccine that is therapeutically effective may vary depending on the particular antigen used, or the condition of the subject, and can be determined by one skilled in the art.

As noted above, the instant invention provides a vaccine composition comprising an antigen component which is an EHV-1 antigen and an adjuvant component, wherein the adjuvant component comprises (or, in different set of embodiments, consists essentially of, or in yet different set of embodiments, consists of) a P-class immunostimulatory oligonucleotide and a SP oil, said oil comprising 2-20% v/v of the vaccine composition.

In a set of embodiments, the vaccine compositions of the instant invention are non-liposomal, the antigen would generally be dispersed or dissolved in the aqueous phase and not enveloped by liposomes or similar structures.

### Antigens

Different antigens derived from EHV are suitable for the instant invention. The antigens may include, without limitation, whole organisms (inactivated, attenuated, and modified live), nucleotides, polynucleotides, peptides, polypeptides, recombinant proteins, synthetic peptides, protein extract, polysaccharides, carbohydrates, fatty acids, teichioc acid, peptidoglycans, lipids, or glycolipids, individually or in any combination thereof.

Live, modified-live, and attenuated viral strains that do not cause disease in a subject have been isolated in non-virulent form or have been attenuated using methods well known in the art, including serial passage in a suitable cell line or exposure to ultraviolet light or a chemical mutagen. Inactivated or killed viral strains are those which have been inactivated by methods known to those skilled in the art, including treatment with formalin, betapropriolactone (BPL), binary ethyleneimine (BEI), sterilizing radiation, heat, or other such methods.

The amount of antigen used to induce an immune response will vary considerably depending on the antigen used, the subject, and the level of response desired, and can be determined by one skilled in the art. For vaccines containing modified live or attenuated EHV, a therapeutically effective amount of the antigen generally ranges from about 10² Tissue Culture Infective Dose (TCID)₅₀ to about 10¹⁰ TCID₅₀, inclusive. For many such viruses, a therapeutically effective dose is generally in the range of about 10² TCID₅₀ to about 10⁸ TCID₅₀, inclusive. In some embodiments, the ranges of therapeutically effective doses are about 10³ TCID₅₀ to about 10⁶ TCID₅₀, inclusive. In some other embodiments, the ranges of therapeutically effective doses are about 10⁴ TCID₅₀ to about 10⁵ TCID₅₀, inclusive.

For vaccines containing inactivated EHV, a therapeutically effective amount of the antigen is generally at least about 100 relative units per dose, and often in the range from about 1,000 to about 4,500 relative units per dose, inclusive. In other embodiments, the therapeutically effective amount of the antigen is in a range from about 250 to about 4,000 relative units per dose, inclusive, from about 500 to about 3,000 relative units per dose, inclusive, from about 750 to about 2,000 relative units per dose, inclusive, or from about 1,000 to about 1,500 relative units per dose, inclusive.

A therapeutically effective amount of antigen in vaccines containing inactivated viruses can also be measured in terms of Relative Potency (RP) per mL. A therapeutically effective amount is often in the range from about 0.1 to about 50 RP per mL, inclusive. In other embodiments, the therapeutically effective amount of the antigen is in a range from about 0.5 to about 30 RP per mL, inclusive, from about 1 to about 25 RP per mL, inclusive, from about 2 to about 20 RP per mL, inclusive, from about 3 to about 15 RP per mL, inclusive, or from about 5 to about 10 RP per mL, inclusive.

The most common varieties of EHV are EHV-1 and EHV-4. EHV is composed of an icosahedral nucleocapsid containing the viral genome, surrounded by an amorphous envelope, which contains eleven glycoproteins (gB, gC, gD, gE, gG, gH, gI, gK, gL, gM and gN). See Paillot et al., Open Vet Sc J, 2008; 2: 68-91.

Suitable non-limiting examples of EHV antigens include whole viruses (inactivated, attenuated, and modified live), protein extracts, envelope proteins and nucleic acid sequences which encode said proteins, as well as recombinant subunit antigens or vectored antigens.

### Saponins and CpGs

Triterpenoid saponins can come from many sources, either plant derived or synthetic equivalents, including but not limited to, Quillaja saponaria, tomatine, ginsing extracts, mushrooms, and an alkaloid glycoside structurally similar to steroidal saponins. Thus, triterpenoids include saponins, squalene, and lanosterol. They are generally used in an amount of about 1 µg to about 5,000 µg per dose. They also are used in an amount of about 1 µg to about 4,000 µg per dose, about 1 µg to about 3,000 µg per dose, about 1 µg to about 2,000 µg per dose, and about 1 µg to about 1,000 µg per dose. They are also used in an amount of about 5 µg to about 750 µg per dose, about 5 µg to about 500 µg per dose, about 5 µg to about 200 µg per dose, about 5 µg to about 100 µg per dose, about 15 µg to about 100 µg per dose, and in an amount of about 30 µg to about 75 µg per dose. If a saponin is used, the adjuvant compositions generally contain an immunologically active saponin fraction from the bark of Quillaja saponaria. The saponin may be, for example, Quil A or another purified or partially purified saponin preparation, which can be obtained commercially. For example, Quil A is sold in the USA by E.M Sergeant Company. QS-7, QS-17, QS-18, and QS-21 may be obtained from Antigenics Company, Massachusetts, USA. Thus, saponin extracts can be used as mixtures or purified individual components such as QS-7, QS-17, QS-18, and QS-21. In one embodiment the Quil A is at least 85% pure. Quil A is at least 90%, 91%, 92%, 93%, 94%, 95%, 96%, 97%, 98%, or 99% pure. CpG oligonucleotides are a recently described class of pharmacotherapeutic agents that are characterized by the presence of an unmethylated CG dinucleotide in specific base-sequence contexts (CpG motif). (Hansel TT, Barnes PJ (eds): New Drugs for Asthma, Allergy and COPD. Prog Respir Res. Basel, Karger, 2001, vol 31, pp 229-232, which is incorporated herein by reference). These CpG motifs are not seen in eukaryotic DNA, in which CG dinucleotides are suppressed and, when present, usually methylated, but are present in bacterial DNA to which they confer immunostimulatory properties.

The adjuvants of the instant invention utilize a so-called P-class immunostimulatory oligonucleotide, more preferably, modified P-class immunostimulatory oligonucleotides. P-class immunostimulatory oligonucleotides are CpG oligonucleotides characterized by the presence of palindromes, generally 6-20 nucleotides long. The P-Class oligonucleotides have the ability to spontaneously self-assemble into concatamers either in vitro and/or in vivo. These oligonucleotides are, in a strict sense, single-stranded, but the presence of palindromes allows for formation of concatamers or possibly stem-and-loop structures. The overall length of P-class immunostimulatory oligonucleotides is between 19 and 100 nucleotides, e.g., 19-30 nucleotides, 30-40 nucleotides, 40-50 nucleotides, 50-60 nucleotides, 60-70 nucleotides, 70-80 nucleotides, 80-90 nucleotides, 90-100 nucleotides.

In one aspect of the invention the immunostimulatory oligonucleotide contains a 5' TLR activation domain and at least two palindromic regions, one palindromic region being a 5' palindromic region of at least 6 nucleotides in length and connected to a 3' palindromic region of at least 8 nucleotides in length either directly or through a spacer.

The P-class immunostimulatory oligonucleotides may be modified according to techniques known in the art. For example, J-modification refers to iodo-modified nucleotides. E-modification refers to ethyl-modified nucleotide(s). Thus, E-modified P-class immunostimulatory oligonucleotides are P-class immunostimulatory oligonucleotides, wherein at least one nucleotide (preferably 5' nucleotide) is ethylated. Additional modifications include attachment of 6-nitro-benzimidazol, O-Methylation, modification with proynyl-dU, inosine modification, 2-bromovinyl attachment (preferably to uridine).

The P-class immunostimulatory oligonucleotides may also contain a modified internucleotide linkage including, without limitations, phosphodiester linkages and phosphorothioate linkages. The oligonucleotides of the instant invention may be synthesized or obtained from commercial sources.

P-Class oligonucleotides and modified P-class oligonucleotides are further disclosed in published PCT application no. WO2008/068638, published on Jun. 12, 2008. Suitable non-limiting examples of modified P-class immunostimulatory oligonucleotides are provided below ("*" refers to a phosphorothioate bond and "_" refers to a phosphodiester bond).

| | |
|---|---|
| SEQ ID NO: 1 | 5' T*C-G*T*C-G*A*C-G*A*T*C-G*G*C*G*C-G*C*G*C*C*G 3' |
| SEQ ID NO: 2 | 5' T*C-G*A*C*G*T*C*G*A*T*C*G*G*C*G*C*G*C*G*C*C*G 3' |
| SEQ ID NO: 3 | 5' T*C*G*A*C*G*T*C*G*A*T*C*G*G*C*G*C*G*C*G*C*C*G*T 3' |
| SEQ ID NO: 4 | 5' JU*C-G*A*C*G*T*C*G*A*T*C*G*G*C*G*C*G*C*G*C*C*G 3' |
| SEQ ID NO: 5 | 5' JU*C-G*A*C*G*T*C*G*A*T*C*G*G*C*G*C*G*C*G*C*C* G*T 3' |
| SEQ ID NO: 6 | 5' JU*C*G*A*C*G*T*C*G*A*T*C*G*G*C*G*C*G*C*G*C*C* G*T 3' |
| SEQ ID NO: 7 | 5' EU*C-G*A*C*G*T*C*G*A*T*C*G*G*C*G*C*G*C*G*C*C*G 3' |
| SEQ ID NO: 8 | 5' JU*C-G*T*C*G*A*C*G*A*T*C*G*G*C*G*G*C*C*G*C*C* G*T 3' |
| SEQ ID NO: 9 | 5' JU*C*G*T*C*G*A*C*G*A*T*C*G*G*C*G*G*C*C*G*C*C* G*T 3' |
| SEQ ID NO: 10 | 5' T*C_G*T*C_G*A*C_G*A*T*C_G*G*C*G*C_G*C*G*C*C*G 3'. |

The amount of P-class immunostimulatory oligonucleotide for use in the adjuvant compositions depends upon the nature of the P-class immunostimulatory oligonucleotide used and the intended species. However, they are generally used in an amount of about 20 µg to about 500 µg per ml. They also are used in an amount of about 25 µg to about 400 mg per ml, about 40 µg to about 250 µg per ml, about 50 µg to about 200 µg per ml, about 100 g per ml to about 200 µg per ml.

### Sterols

Sterols include β-sitosterol, stigmasterol, ergosterol, ergocalciferol, and cholesterol. These sterols are well known in the art and can be purchased commercially. For example, cholesterol is disclosed in the Merck Index, 12th Ed., p. 369. The amount of sterols suitable for use in the adjuvant compositions depends upon the nature of the sterol used. However, they are generally used in an amount of about 1 µg to about 5,000 µg per ml. They also are used in an amount of about 1 µg to about 4,000 µg per ml, about 1 µg to about 3,000 µg per ml, about 1 µg to about 2,000 µg per ml, and about 1 µg to about 1,000 µg per ml. They are also used in an amount of about 5 µg to about 750 µg per ml, about 5 µg to about 500 µg per ml, about 5 µg to about 200 µg per ml, about 5 µg to about 100 µg per ml, about 15 µg to about 100 µg per ml, and about 30 µg to about 75 µg per ml.

The preparation of the compositions containing the saponin and the sterol according to Example 1 below is within the ordinary skill in the art. Briefly, an aqueous mixture is prepared, said mixture comprising the antigen, the P-class immunomodulatory oligonucleotide, and the saponin. The sterol is then gradually (or dropwise) added to that mixture.

### Oily Phase

The adjuvant component of the present invention comprises a SP oil in the amount of about 2 to about 20% v/v of the vaccine composition, e.g., about 3 to about 15%, about 5 to about 15%, about 10 to about 15%, about 10 to about 20 % etc. The oily phase comprises about 2 to about 20% v/v SP oil and optionally comprises one or more emulsifiers. In one alternative the oily phase comprises two emulsifiers, one of which is lipid-soluble and the other is water-soluble.

Multiple oils and combinations thereof are also disclosed herein. These oils include, animal oils, vegetable oils, as well as non-metabolizable oils. Examples of vegetable oils are corn oil, peanut oil, soybean oil, coconut oil, and olive oil. An example of animal oils is squalane. Examples of non-metabolizable oils include light mineral oil, straight chained or branched saturated oils, squalane and the like.

The oil is SP oil, which comprises a polyoxyethylene-polyoxypropylene block copolymer (available from BASF, Mt. Olive, NJ), squalane (available from Kodak, Rochester, NY), and polyoxyethylene sorbitan monooleate (TWEEN®80, available from Sigma Chemical, St. Louis, MO). Squalane is used at approximately 5% (w/v), polyoxyethylene-polyoxypropylene block copolymer is used at approximately 2.5% (w/v), polyoxyethylene sorbitan monooleate (TWEEN®-80), is used at approximately 0.2%(w/v) prepared in a pharmaceutically acceptable diluent of either buffer, water, normal saline or cell culture growth media.

Emulsifiers suitable for use in the present emulsions include natural biologically compatible emulsifiers and non-natural synthetic surfactants. Biologically compatible emulsifiers include phospholipid compounds or a mixture of phospholipids. Preferred phospholipids are phosphatidylcholines (lecithin), such as soy or egg lecithin. Lecithin can be obtained as a mixture of phosphatides and triglycerides by water-washing crude vegetable oils, and separating and drying the resulting hydrated gums. A refined product can be obtained by fractionating the mixture for acetone insoluble phospholipids and glycolipids remaining after removal of the triglycerides and vegetable oil by acetone washing. Alternatively, lecithin can be obtained from various commercial sources. Other suitable phospholipids include phosphatidylglycerol, phosphatidylinositol, phosphatidylserine, phosphatidic acid, cardiolipin, and phosphatidylethanolamine. The phospholipids may be isolated from natural sources or conventionally synthesized.

Alternately, emulsifiers used herein do not include lecithin, or use lecithin in an amount which is not immunologically effective.

Non-natural, synthetic emulsifiers suitable for use in the adjuvant formulations of the present invention include sorbitan-based non-ionic surfactants, e.g. fatty-acid-substituted sorbitan surfactants (commercially available under the name SPAN® or ARLACEL®), fatty acid esters of polyethoxylated sorbitol (TWEEN®), polyethylene glycol esters of fatty acids from sources such as castor oil (EMULFOR®); polyethoxylated fatty acid (e.g., stearic acid available under the name SIMULSOL M-53), polyethoxylated isooctylphenol/formaldehyde polymer (TYLOXAPOL®), polyoxyethylene fatty alcohol ethers (BRIJ®); polyoxyethylene nonphenyl ethers (TRITON® N), polyoxyethylene isooctylphenyl ethers (TRITON® X). Preferred synthetic surfactants are the surfactants available under the name SPAN® and TWEEN®, such as TWEEN-80 (Polyoxyethylene (20) sorbitan monooleate) and SPAN-80 (sorbitan monooleate).

Generally speaking, the emulsifier(s) may be present in the vaccine composition in an amount of 0.01% to 10% by volume, preferably, 0.1% to 5%, more preferably 1% to 3%.

Preparation of the vaccine compositions comprising the SP oil is straightforward and entails mixing the aqueous phase comprising the antigen, the P-class immunostimulatory oligonucleotide, and, optionally, a water-soluble emulsifier, with the SP oil and, optionally, a lipid-soluble emulsifier. The resulting mixture is emulsified.

Other components of the compositions can include pharmaceutically acceptable excipients, such as carriers, solvents, and diluents, isotonic agents, buffering agents, stabilizers, preservatives, vaso-constrictive agents, antibacterial agents, antifungal agents, and the like. Typical carriers, solvents, and diluents include water, saline, dextrose, ethanol, glycerol, oil, and the like. Representative isotonic agents include sodium chloride, dextrose, mannitol, sorbitol, lactose, and the like. Useful stabilizers include gelatin, albumin, and the like.

As used herein, "a pharmaceutically-acceptable carrier" includes any and all solvents, dispersion media, coatings, adjuvants, stabilizing agents, diluents, preservatives, antibacterial and antifungal agents, isotonic agents, adsorption delaying agents, and the like. The carrier(s) must be "acceptable" in the sense of being compatible with the other components of the compositions and not deleterious to the subject. Typically, the carriers will be sterile and pyrogen-free, and selected based on the mode of administration to be used. It is well known by those skilled in the art that the preferred formulations for the pharmaceutically acceptable carrier which comprise the compositions are those pharmaceutical carriers approved in the applicable regulations promulgated by the United States (US) Department of Agriculture or US Food and Drug Administration, or equivalent government agency in a non-US country. Therefore, the pharmaceutically accepted carrier for commercial production of the compositions is a carrier that is already approved or will be approved by the appropriate government agency in the US or foreign country.

The compositions optionally can include compatible pharmaceutically acceptable (i.e., sterile or non-toxic) liquid, semisolid, or solid diluents that serve as pharmaceutical vehicles, excipients, or media. Diluents can include water, saline, dextrose, ethanol, glycerol, and the like. Isotonic agents can include sodium chloride, dextrose, mannitol, sorbitol, and lactose, among others. Stabilizers include albumin, among others.

The compositions can also contain antibiotics or preservatives, including, for example, gentamicin, merthiolate, or chlorocresol. The various classes of antibiotics or preservatives from which to select are well known to the skilled artisan.

### Administration and Use of the Compositions

Dose sizes of the compositions typically range from about 0.5 mL to about 5 mL, inclusive, depending on the subject and the antigen. For example, for a canine or feline, a dose of about 0.5 to about 1 mL is typically used, while in larger animals a dose of about 1-5 mL is typically used. However, these adjuvants also can be formulated in microdoses, wherein doses of about 100 to about 500 µL can be used.

The routes of administration for the adjuvant compositions include, without limitations, subcutaneous, intramuscular, parenteral, oral, oronasal, intranasal, intratracheal, topical, etc. Any suitable device may be used to administer the compositions, including syringes, droppers, needleless injection devices, patches, and the like. The route and device selected for use will depend on the composition of the adjuvant, the antigen, and the subject, and such are well known to the skilled artisan.

The invention will be further described in the following non-limiting examples.

### EXAMPLE

### Example 1: EHV antigen

### Animals

Cross-bred intact male and female equine, age 11-12 months, were divided into treatment groups containing 5 horses per group. Horses were maintained in outdoor facilities during the vaccination phase. Since nearly all horses are exposed to EHV early in life, horses are not truly naive. SN titers are used to select animals that have low titers however previous exposure may impact interpretation of test results. Conventional killed EHV-1 was used as antigen in this study.

The experimental design is provided in Table 4.

**Table 4: Experimental Design for EHV Antigen Study in Horses**

| **Group (N=5)** | **Antigen** | **Adjuvant** | | **No. Doses & Days** | **Route & Vol./Dose** |
|---|---|---|---|---|---|
| | | **1** | **2** | | |
| T01 | PBS | N/A | N/A | 2 doses Days: 0 & 21 | IM 1ml |
| T02 | EHV Antigen | --- | 5% SP Oil | | |
| T03 | EHV Antigen | P-CpG (SEQ ID NO: 8; 100 µg /dose) | QuilA (50µg) Cholesterol (50µg) | | |
| T04 | EHV Antigen | P-CpG (SEQ ID NO: 8; 100 µg /dose) | 5% SP oil | | |

The results are summarized in the tables below.

**Table 5: PBMC ELISPot Stimulation Index Results for EHV Antigen Study in Horses**

| Group | **EHV1 Ag - Stimulation Index PBMCs on Day:** | | | |
|---|---|---|---|---|
| | **0** | **7** | **28** | **49** |
| T01 | 9 | 72 | 40 | 23 |
| T02 | 7 | 19 | 25 | 26 |
| T03 | 4 | 14 | 54 | 89 |
| T04 | 8 | 15 | 11 | 45 |

**Table 6: PBMC ELISPot SFCs/10⁶ PBMCs Results for EHV Antigen Study in Horses**

| Group | **EHV1 Ag - SFCs/10⁶ PBMCs on Day:** | | | |
|---|---|---|---|---|
| | **0** | **7** | **28** | **49** |
| T01 | 195 | 281 | 292 | 299 |
| T02 | 79 | 156 | 431 | 338 |
| T03 | 122 | 345 | 982 | 951 |
| T04 | 126 | 174 | 561 | 814 |

**Table 7: Serum Neutralizing Antibody Titer Results for EHV Antigen Study in Horses**

| **Group** | **Serum Neutralizing Antibody Titer on Day:** | | | | |
|---|---|---|---|---|---|
| | **0** | **7** | **21** | **28** | **35** |
| T01 | 7.9 | 12.2 | 8.5 | 24.6 | 16.0 |
| T02 | 10.6 | 30.7 | 18.8 | 36.5 | 21.4 |
| T03 | 13.5 | 32.7 | 24.8 | 72.6 | 50.3 |
| T04 | 7.2 | 47.1 | 46.1 | 94.8 | 60.4 |

**Table 8: Summary Results Table for Reactivity Post-Second Vaccination for EHV Antigen Study in Horses (Day 29)**

| Assay | T01 | T02 | T03 | T04 |
|---|---|---|---|---|
| IFN g ELISpot | - | - | ++++ | +++ |
| IL-2 RT-PCR | ++ | ++ | ++ | +++ |
| IL-4 ELISpot SFC | - | - | +++ | ++ |
| IL-10 ELISA | - | - | ++ | ++ |
| GRZ B RT-PCR | - | - | + | + |
| SN Titers | - | + | ++ | +++ |

| | | | | |
|---|---|---|---|---|
| - no response + Below Average Response ++ Average Response +++ Above Average Response ++++ Significantly Above Average Response | | | | |

These results demonstrate that P-class oligonucleotides provide strong adjuvanting effects in combination with SP Oil.

Although the invention herein has been described with reference to particular embodiments, it is to be understood that these embodiments are merely illustrative of the principles and applications of the present invention. It is therefore to be understood that numerous modifications may be made to the illustrative embodiments and that the scope of the present invention is defined by the following claims.

## Claims

1. A vaccine composition comprising an equine herpes virus (EHV) antigen component and an adjuvant component, wherein the antigen component comprises an EHV-1 antigen and the adjuvant component comprises a P-class CpG oligonucleotide and SP oil, wherein the SP oil is present in the amount of 2-20% v/v and the SP oil comprises about 2.5% w/v polyoxyethylene-polyoxypropylene block copolymer, about 5% w/v squalane, and about 0.2% w/v polyoxyethylene sorbitan monooleate.

2. A vaccine composition of claim 1, wherein the adjuvant consists essentially of the P-class CpG oligonucleotide and SP oil.

3. The vaccine composition of claim 1 or 2, which is a non-liposomal composition.

4. The vaccine composition of any one of claims 1-3, wherein SP oil is present in the amount of 5-10% v/v of the vaccine composition.

5. The vaccine composition of any one of claims 1-4 wherein SP oil present in the amount of 10% v/v of the composition.

6. The vaccine composition of any one of claims 1-5, wherein said P-class CpG oligonucleotide is present in the amount of 20-500 ug/ml.

7. The vaccine composition of claim 6, wherein said P-class CpG oligonucleotide is present in the amount of 100-250 ug/mL

8. The vaccine composition of any one of claims 1-7, wherein said P-class CpG oligonucleotide is modified.

9. The vaccine composition of claim 8, wherein said modified P-class CpG oligonucleotide is JU-modified or E-modified.

10. The vaccine composition of claim 9, wherein said modified P-class CpG oligonucleotide is SEQ ID NO: 7, SEQ ID NO: 8 or SEQ ID NO: 9.

## Patentansprüche

1. Impfstoffzusammensetzung, umfassend eine Pferdeherpesvirus (Equine Herpes Virus, EHV)-Antigenkomponente und eine Adjuvanskomponente, wobei die Antigenkomponente ein EHV-1-Antigen umfasst, und die Adjuvanskomponente ein CpG-Oligonukleotid der P-Klasse und SP-Öl umfasst, wobei das SP-Öl in einer Menge von 2 bis 20% V/V vorhanden ist und das SP-Öl etwa 2,5% G/V Polyoxyethylen-Polyoxypropylen-Blockcopolymer, etwa 5% G/V Squalan und etwa 0,2% G/V Polyoxyethylen-Sorbitanmonooleat umfasst.

2. Impfstoffzusammensetzung nach Anspruch 1, wobei das Adjuvans im Wesentlichen aus dem CpG-Oligonukleotid der P-Klasse und SP-Öl besteht.

3. Impfstoffzusammensetzung nach Anspruch 1 oder 2, die eine nicht-liposomale Zusammensetzung ist.

4. Impfstoffzusammensetzung nach einem der Ansprüche 1-3, wobei SP-Öl in einer Menge von 5 bis 10% V/V der Impfstoffzusammensetzung vorhanden ist.

5. Impfstoffzusammensetzung nach einem der Ansprüche 1-4, wobei SP-Öl in der Menge von 10% V/V der Zusammensetzung vorhanden ist.

6. Impfstoffzusammensetzung nach einem der Ansprüche 1-5, wobei das CpG-Oligonukleotid der P-Klasse in einer Menge von 20-500 µg/ml vorhanden ist.

7. Impfstoffzusammensetzung nach Anspruch 6, wobei das CpG-Oligonukleotid der P-Klasse in einer Menge von 100-250 µg/ml vorhanden ist.

8. Impfstoffzusammensetzung nach einem der Ansprüche 1-7, wobei das CpG-Oligonukleotid der P-Klasse modifiziert ist.

9. Impfstoffzusammensetzung nach Anspruch 8, wobei das modifizierte CpG-Oligonukleotid der P-Klasse JU-modifiziert oder E-modifiziert ist.

10. Impfstoffzusammensetzung nach Anspruch 9, wobei das modifizierte CpG-Oligonukleotid der P-Klasse SEQ ID NO: 7, SEQ ID NO: 8 oder SEQ ID NO: 9 ist.

## Revendications

1. Composition de vaccin comprenant un composant antigène d'herpèsvirus équin (HVE) et un composant adjuvant, dans laquelle le composant antigène comprend un antigène de HVE-1 et le composant adjuvant comprend un oligonucléotide CpG de classe P et une huile SP, dans laquelle l'huile SP est présente en une quantité de 2 à 20 % v/v et l'huile SP comprend environ 2,5 % p/v de copolymère séquencé de polyoxyéthylène-polyoxypropylène, environ 5 % p/v de squalane, et environ 0,2 % p/v de monooléate de sorbitan polyoxyéthyléné.

2. Composition de vaccin selon la revendication 1, dans laquelle l'adjuvant est constitué essentiellement de l'oligonucléotide CpG de classe P et de l'huile SP.

3. Composition de vaccin selon la revendication 1 ou 2, qui est une composition non liposomale.

4. Composition de vaccin selon l'une quelconque des revendications 1 à 3, dans laquelle l'huile SP est présente en une quantité de 5 à 10 % v/v de la composition de vaccin.

5. Composition de vaccin selon l'une quelconque des revendications 1 à 4, dans laquelle l'huile SP est présente en une quantité de 10 % v/v de la composition.

6. Composition de vaccin selon l'une quelconque des revendications 1 à 5, dans laquelle ledit oligonucléotide CpG de classe P est présent en une quantité de 20 à 500 µg/ml.

7. Composition de vaccin selon la revendication 6, dans laquelle ledit oligonucléotide CpG de classe P est présent en une quantité de 100 à 250 µg/ml.

8. Composition de vaccin selon l'une quelconque des revendications 1 à 7, dans laquelle ledit oligonucléotide CpG de classe P est modifié.

9. Composition de vaccin selon la revendication 8, dans laquelle ledit oligonucléotide CpG de classe P est JU-modifié ou E-modifié.

10. Composition de vaccin selon la revendication 9, dans laquelle ledit oligonucléotide CpG de classe P est la SEQ ID NO : 7, SEQ ID NO : 8 ou SEQ ID NO : 9.


### SEQUENCE LISTING

<110> ZOETIS LLC DOMINOWSKI, PAUL JOSEPH RAI, SHARATH K. SLY, LAUREL MARY COOK, COREY PATRICK MWANGI, DUNCAN FOSS, DENNIS L. KREBS, RICHARD LEE
<120> NOVEL VACCINE COMPOSITIONS COMPRISING IMMUNOSTIMULATORY OLIGONUCLEOTIDES
<130> ZP000011A
<150> US 61/823189
   <151> 2013-05-14
<160> 10
<170> PatentIn version 3.5
<210> 1
   <211> 23
   <212> DNA
   <213> Artificial Sequence
<220>
   <223> CpG oligonucleotide
<400> 1
   tcgtcgacga tcggcgcgcg ccg 23
<210> 2
   <211> 23
   <212> DNA
   <213> Artificial Sequence
<220>
   <223> CpG oligonucleotide
<400> 2
   tcgacgtcga tcggcgcgcg ccg 23
<210> 3
   <211> 24
   <212> DNA
   <213> Artificial Sequence
<220>
   <223> CpG oligonucleotide
<400> 3
   tcgacgtcga tcggcgcgcg ccgt 24
<210> 4
   <211> 23
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 4
   tcgacgtcga tcggcgcgcg ccg 23
<210> 5
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 5
   tcgacgtcga tcggcgcgcg ccgt 24
<210> 6
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 6
   tcgacgtcga tcggcgcgcg ccgt 24
<210> 7
   <211> 23
   <212> DNA
   <213> Artificial Sequence
<220>
   <223> CpG oligonucleotide
<220>
   <221> modified_base
   <222> (1)..(1)
   <223> 5'-Ethyl-2'-deoxyuridine
<400> 7
   tcgacgtcga tcggcgcgcg ccg 23
<210> 8
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 8
   tcgtcgacga tcggcggccg ccgt 24
<210> 9
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 9
   tcgtcgacga tcggcggccg ccgt 24
<210> 10
   <211> 23
   <212> DNA
   <213> Artificial Sequence
<220>
   <223> CpG oligonucleotide
<400> 10
   tcgtcgacga tcggcgcgcg ccg 23